# EUROPEAN PATENT SPECIFICATION

(11) **EP 2 754 487 B1**
(45) Date of publication and mention of the grant of the patent: **06.02.2019**
(21) Application number: 12829878.3
(22) Date of filing: 05.09.2012
(51) Int. Cl.: B01J 19/10

(54) **ULTRASONIC OSCILLATOR**
ULTRASCHALLOSZILLATOR
OSCILLATEUR ULTRASONORE

(30) Priority: 06.09.2011 US 201161531438 P
(43) Date of publication of application: 16.07.2014
(73) Proprietor: Huang, Lynn, L. H., Tainan City, Taiwan (TW)
(72) Inventor: Huang, Lynn, L. H., Tainan City, Taiwan (TW)
(74) Representative: Viering, Jentschura & Partner mbB Patent- und Rechtsanwälte
(86) International application number: PCT/CN2012/001242
(87) International publication number: WO 2013/033973

(56) References cited:
- WO-A2-2005/058480
- CH-A- 430 291
- CN-A- 1 398 663
- CN-A- 1 411 899
- CN-Y- 2 581 058
- CN-Y- 201 186 212
- CN-Y- 201 394 458
- US-A- 2 941 908
- US-A- 5 484 202

## Description

### 1. Field of the Invention

The present invention relates to an ultrasonic oscillator, especially to an ultrasonic oscillator for manufacturing porous collagen matrices.

### 2. Description of the Prior Arts

Collagen is widely used in modern society. Biotechnology industry endeavors to manufacture collagen faster with good quality. Therefore, manufacturing porous collagen matrices from connective tissues accordingly becomes an important issue.

The main objective of the present invention is to provide an ultrasonic oscillator for manufacturing porous collagen matrices.

To achieve aforementioned objective, the present invention develops an ultrasonic oscillator comprising:
a hollow body;
a receiving groove mounted securely in the body;
a transmission component mounted through the body, and having
a hydraulic cylinder mounted on the top of the body;
a cover connected to the hydraulic cylinder and selectively sealing the receiving groove; and
multiple supporting rods for providing vibration, and mounted through the top of the body and the cover, and connected to the hydraulic cylinder;
multiple frames connecting to the transmission component, respectively hung on the supporting rods, and selectively mounted in the receiving groove by the transmission component, and each frame having multiple containers inserted therein;
multiple oscillation devices connecting to and selectively vibrating a corresponding frame; and
an ultrasonic mechanism mounted in the body.

The ultrasonic oscillator as described, wherein the transmission component comprises a hydraulic cylinder mounted on the top of the body; a cover connected to the hydraulic cylinder and selectively sealing the receiving groove; and multiple supporting rods mounted vibratile through the top of the body and the cover and connected to the hydraulic cylinder; and the frames are respectively hung on the supporting rods.

The ultrasonic oscillator as described, wherein each oscillation device comprises a spur gear mounted securely on the corresponding supporting rod; a motor mounted on the body; and an eccentric gear driven by the motor and selectively engaging the spur gear.

The ultrasonic oscillator as described further comprising a vacuum pump mounted on the cover, wherein the cover has a pressure relief device mounted thereon.

The ultrasonic oscillator as described further comprising a vacuum pump mounted on the cover, wherein the cover has a pressure relief device mounted thereon.

The ultrasonic oscillator as described further comprising a spraying mechanism mounted in the receiving groove, wherein the receiving groove has a discharging pipe mounted on a bottom of the receiving groove and protruding out of the body.

The present invention has following advantages. With the cooperation between the oscillation devices and the ultrasonic mechanism, the connective tissues in the containers gradually become porous collagen matrices to be provided for the needs of the industry.

### IN THE DRAWINGS

Fig. 1 is an operational front view of an ultrasonic oscillator for manufacturing porous collagen matrices, showing the frames being moved into the receiving groove;
Fig. 2 is an enlarged side view of a container of the ultrasonic oscillator in Fig. 1;
Fig. 3 is an operational front view of the ultrasonic oscillator in Fig. 1, showing the frames oscillating; and
Fig. 4 is another operational front view of the ultrasonic oscillator in Fig. 1, showing the frames oscillating.

With the drawings and the preferred embodiment in accordance with the present invention as follow, the means to achieve the purpose of the present invention will be understood and appreciated more fully.

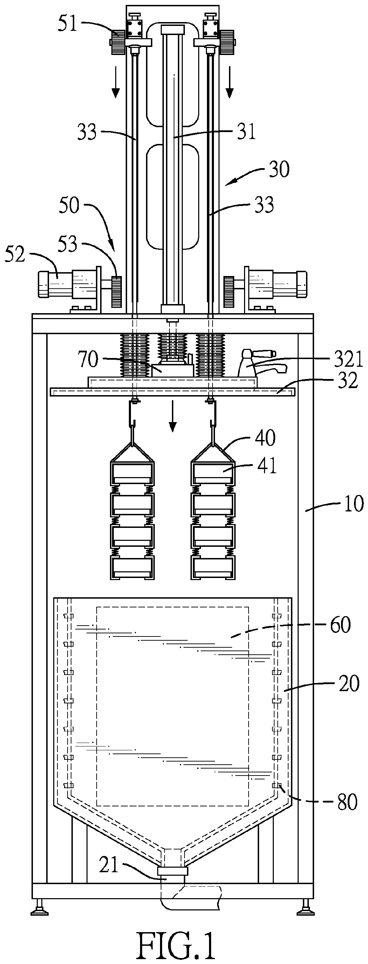

With reference to Fig. 1, an ultrasonic oscillator in accordance with the present invention comprises a hollow body 10, a receiving groove 20, a transmission component 30, multiple frames 40, multiple oscillation devices 50, an ultrasonic mechanism 60, a vacuum pump 70 and a spraying mechanism 80.

The receiving groove 20 is mounted securely in the body 10 and is mounted near a bottom of the body 10. The receiving groove 20 has a discharging pipe 21 mounted on a bottom of the receiving groove 20 and protruding out of the body 10.

The transmission component 30 is mounted through a top of the body 10 and comprises a hydraulic cylinder 31, a cover 32 and multiple supporting rods 33. The hydraulic cylinder 31 is mounted on the top of the body 10. The cover 32 is connected to the hydraulic cylinder 31, selectively seals the receiving groove 20 and has a pressure relief device 321 mounted thereon. The supporting rods 33 are mounted vibratile through the top of the body 10 and the cover 32 and are connected to the hydraulic cylinder 31.

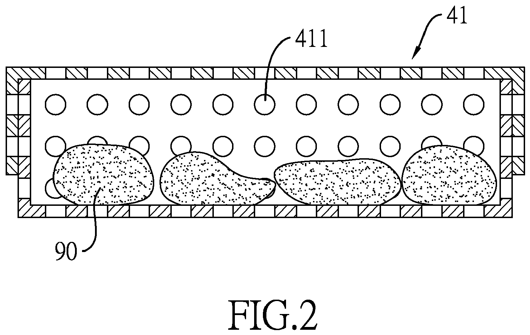

With reference to Figs. 1 and 2, the frames 40 connect to the transmission component 30 and are selectively mounted in the receiving groove 20. In a preferred embodiment, the frames 40 are respectively hung on the supporting rods 33. Each frame 40 has multiple containers 41 inserted therein. Each container 41 has multiple holes 411 formed therethrough.

Each oscillation device 50 connects to and selectively vibrates a corresponding frame 40 and comprises a spur gear 51, a motor 52 and an eccentric gear 53. The spur gear 51 is mounted securely on the corresponding supporting rod 33. The motor 52 is mounted on the body 10. The eccentric gear 53 is driven by the motor 52 and selectively engages the spur gear 51.

The ultrasonic mechanism 60 is mounted in the body 10.

The vacuum pump 70 is mounted on the cover 32 and selectively extracts the air in the receiving groove 20 to create vacuum in the receiving groove 20.

The spraying mechanism 80 is mounted in the receiving groove 20 and selectively sprays water to moisten the subjects in the containers 41. The water sprayed into the receiving groove 20 is discharged out from the discharging pipe 21. The spraying mechanism 80 may comprise multiple nozzles.

With reference to Figs. 1 and 2, connective tissues 90 are put in the containers 41. The hydraulic cylinder 31 pushes the cover 32 to move downward and seals the receiving groove 20 and the frames 40 are also moved into the receiving groove 20. The vacuum pump 70 may create vacuum in the receiving groove 20 when the cover 32 seals the receiving groove 20.

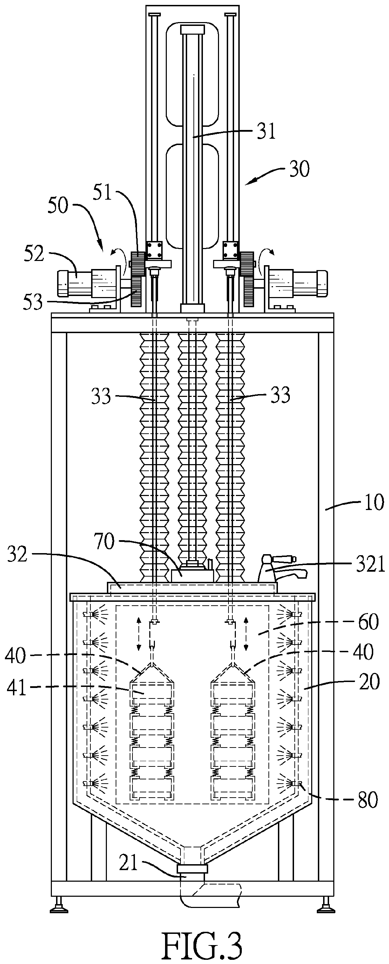

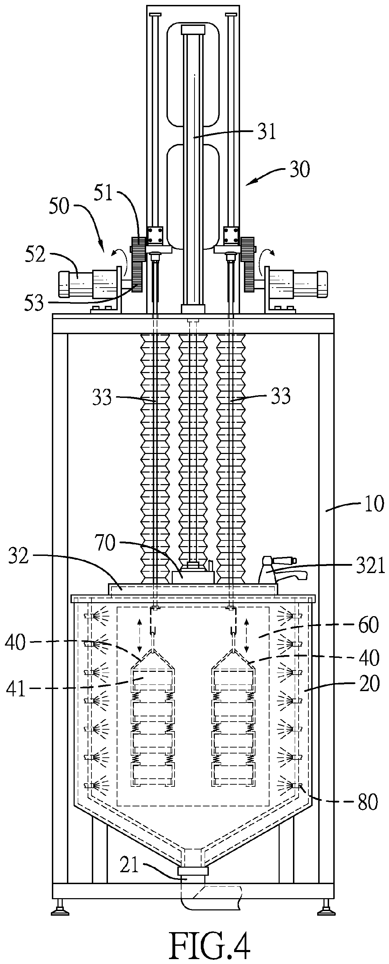

With reference to Figs. 3 and 4, the eccentric gears engage the spur gears 51. The motors 52 operate and the eccentric gears 53 are rotated to drive the spur gears 51 to rotate and oscillate. Therefore, the supporting rods 33 oscillate to vibrate the frames 40 and containers 41. The ultrasonic mechanism 60 and the spraying mechanism 80 also operate. Then the connective tissues in the containers 41 gradually become porous collagen matrices.

When the manufacturing process is finished, the pressure relief device 321 is operated to allow the air to infuse into the receiving groove 20. Then the hydraulic cylinder 31 pulls the cover 32 and the frames 40 to move upward and leave the receiving groove 20. Then the manufacturer may take the containers 41 out of the frames 40.

Even though numerous characteristics and advantages of the present invention have been set forth in the foregoing description, together with details of the structure and features of the invention, the disclosure is illustrative only. Changes may be made in the details, especially in matters of shape, size, and arrangement of parts within the principles of the invention to the full extent indicated by the broad general meaning of the terms in which the appended claims are expressed.

## Claims

1. An ultrasonic oscillator comprising:
a hollow body (10);
a receiving groove (20) mounted securely in the body (10);
a transmission component (30) mounted through the body (10), and having a hydraulic cylinder (31) mounted on the top of the body (10);
a cover (32) connected to the hydraulic cylinder (31) and selectively sealing the receiving groove (20); and
multiple supporting rods (33) for providing vibration, and
mounted through the top of the body (10) and the cover (32), and connected to the hydraulic cylinder (31);
multiple frames (40) connecting to the transmission component (30), respectively hung on the supporting rods (33), and selectively mounted in the receiving groove (20) by the transmission component (30), and each frame (40) having multiple containers (41) inserted therein;
multiple oscillation devices (50) connecting to and sclcctivcly vibrating a corresponding frame (40); and
an ultrasonic mechanism (60) mounted in the receiving groove (20).

2. The ultrasonic oscillator as claimed in claim 1, wherein each oscillation device (50) comprises a spur gear (51) mounted securely on the corresponding supporting rod (33); a motor (52) mounted on the body (10); and an eccentric gear (53) driven by the motor (52) and selectively engaging the spur gear (51).

3. The ultrasonic oscillator as claimed in claim 1 or 2 further comprising a vacuum pump (70) mounted on the cover (32), wherein the cover (32) has a pressure relief device (321) mounted thereon.

4. The ultrasonic oscillator as claimed in claim 1 or 2 further comprising a spraying mechanism (80) mounted in the receiving groove (20), wherein the receiving groove (20) has a discharging pipe (21) mounted on a bottom of the receiving groove (20) and protruding out of the body (10).

5. The ultrasonic oscillator as claimed in claim 3 further comprising a spraying mechanism (80) mounted in the receiving groove (20), wherein the receiving groove (20) has a discharging pipe (21) mounted on a bottom of the receiving groove (20) and protruding out of the body (10).

## Patentansprüche

1. Ultraschalloszillator aufweisend:
einen hohlen Körper (10);
eine Empfangsnut (20), die sicher in dem Körper (10) angebracht ist;
eine Transmissionskomponente (30), die durch den Körper (10) angebracht ist, und die einen hydraulischen Zylinder (31) aufweist, der an der Oberseite des Körpers (10) angebracht ist;
eine Abdeckung (32), die mit dem hydraulischen Zylinder (31) verbunden ist, und die Empfangsnut (20) selektiv abdichtet; und
mehrere Tragstäbe (33) zum Bereitstellen von Vibration, und angebracht durch die Oberseite des Körpers (10) und die Abdeckung (32), und verbunden mit dem hydraulischen Zylinder (31);
mehrere Rahmen (40) verbunden mit der Transmissionskomponente (30), jeweils an den Tragstäben (33) aufgehängt, und selektiv angebracht in der Empfangsnut (20) durch die Transmissionskomponente (30), wobei jeder Rahmen (40) mehrere Container (41) aufweist, die darin eingebracht sind;
mehrere Oszillationsvorrichtungen (50), verbunden mit und selektiv vibrierend einen entsprechenden Rahmen (40); und
einen Ultraschallmechanismus (60) angebracht in der Empfangsnut (20).

2. Der Ultraschalloszillator gemäß Anspruch 1, wobei jede Oszillationsvorrichtung (50) ein Stirnradgetriebe (51) aufweist, das sicher an dem entsprechenden Tragstab (33) angebracht ist; einen Motor (52), der an dem Körper (10) angebracht ist; und ein exzentrisches Rad (53), das durch den Motor (52) angetrieben wird, und das selektiv mit dem Stirnradgetriebe (51) zusammenwirkt.

3. Der Ultraschalloszillator gemäß Anspruch 1 oder 2, weiter aufweisend eine Vakuumpumpe (70), die auf der Abdeckung (32) angebracht ist, wobei die Abdeckung (32) eine Druckentlastungsvorrichtung (321) aufweist, die darauf angeordnet ist.

4. Der Ultraschalloszillator gemäß Anspruch 1 oder 2, weiter aufweisend einen Sprühmechanismus (80), der in der Empfangsnut (20) angeordnet ist, wobei die Empfangsnut (20) ein Auslassrohr (21) aufweist, das an einer Unterseite der Empfangsnut (20) angebracht ist und sich aus dem Körper (10) heraus erstreckt.

5. Der Ultraschalloszillator gemäß Anspruch 3, weiter aufweisend einen Sprühmechanismus (80), der in der Empfangsnut (20) angeordnet ist, wobei die Empfangsnut (20) ein Auslassrohr (21) aufweist, das an einer Unterseite der Empfangsnut (20) angebracht ist und sich aus dem Körper (10) heraus erstreckt.

## Revendications

1. Oscillateur ultrasonore comprenant :
un corps creux (10) ;
une rainure de réception (20) montée de manière fixe dans le corps (10) ;
un composant de transmission (30) monté à travers le corps (10) et ayant un cylindre hydraulique (31) monté sur le dessus du corps (10) ;
un couvercle (32) raccordé au cylindre hydraulique (31) et scellant sélectivement la rainure de réception (20) ; et
plusieurs tiges de support (33) pour fournir une vibration, et
montées à travers le dessus du corps (10) et le couvercle (32) et raccordées au cylindre hydraulique (31) ;
plusieurs bâtis (40) se raccordant au composant de transmission (30), respectivement suspendus aux tiges de support (33) et sélectivement montés dans la rainure de réception (20) par le composant de transmission (30), et chaque bâti (40) ayant plusieurs récipients (41) insérés à l'intérieur de ce dernier ;
plusieurs dispositifs d'oscillation (50) se raccordant à et faisant sélectivement vibrer un bâti (40) correspondant ; et
un mécanisme ultrasonore (60) monté dans la rainure de réception (20).

2. Oscillateur ultrasonore selon la revendication 1, dans lequel chaque dispositif d'oscillation (50) comprend un engrenage cylindrique (51) monté, de manière fixe, sur la tige de support (33) correspondante; un moteur (52) monté sur le corps (10) ; et un engrenage excentrique (53) entraîné par le moteur (52) et mettant sélectivement en prise l'engrenage cylindrique (51).

3. Oscillateur ultrasonore selon la revendication 1 ou 2, comprenant en outre une pompe à vide (70) montée sur le couvercle (32), dans lequel le couvercle (32) a un dispositif de décharge de pression (321) monté sur ce dernier.

4. Oscillateur ultrasonore selon la revendication 1 ou 2, comprenant en outre un mécanisme de pulvérisation (80) monté dans la rainure de réception (20), dans lequel la rainure de réception (20) a un tuyau de décharge (21) monté sur un fond de la rainure de réception (20) et faisant saillie du corps (10).

5. Oscillateur ultrasonore selon la revendication 3, comprenant en outre un mécanisme de pulvérisation (80) monté dans la rainure de réception (20), dans lequel la rainure de réception (20) a un tuyau de décharge (21) monté sur un fond de la rainure de réception (20) et faisant saillie du corps (10).
